# EUROPEAN PATENT SPECIFICATION

(11) **EP 2 626 060 B1**
(45) Date of publication and mention of the grant of the patent: **22.05.2019**
(21) Application number: 11830730.5
(22) Date of filing: 06.10.2011
(51) Int. Cl.: A61K 8/23, A61K 8/25, A61K 8/26, A61K 8/37, A61K 8/81, A61Q 1/12, A61K 8/27, A61K 8/29, A61Q 1/02, A61K 8/02

(54) **SOLID POWDER COSMETIC**
FESTPULVERKOSMETIKUM
POUDRE COSMÉTIQUE SOLIDE

(30) Priority: 06.10.2010 JP 2010226862
(43) Date of publication of application: 14.08.2013
(73) Proprietor: Shiseido Company, Ltd., Chuo-ku Tokyo 104-8010 (JP)
(72) Inventor: SHIRAO Sachiko, Yokohama-shi Kanagawa 224-8558 (JP); NARUMI Yuuko, Yokohama-shi Kanagawa 224-8558 (JP)
(74) Representative: Schön, Christoph
(86) International application number: PCT/JP2011/073074
(87) International publication number: WO 2012/046798

(56) References cited:
- EP-A1- 1 481 659
- EP-A1- 1 803 750
- EP-A1- 2 233 126
- JP-A- 9 227 792
- JP-A- 10 087 433
- JP-A- 2003 342 127
- JP-A- 2010 030 952
- JP-A- 2010 163 389
- US-A1- 2005 118 220
- US-B1- 6 242 092

## Description

### Technical Field

The present invention relates to a cosmetic composition and, more particularly, to a solid powder cosmetic composition used as a make-up cosmetic composition such as a foundation.

### Background Art

Make-up cosmetic compositions have long been used for their effects of making users more beautiful (aesthetic effect), protecting skin (protective effect), and producing various positive moods of users (psychological effect). In light of usability, function, seasonability, etc. of make-up cosmetic compositions, new types of such compositions adapted to the needs of the times have been developed one after another.

Currently, one purpose of using a make-up cosmetic composition is to suppress "skin dullness." For example, in order to suppress "skin dullness," a basic cosmetic product which can activate dermal blood flow is used, or massage is applied to the skin. Make-up cosmetic compositions are actively used for masking "dullness" appearing on the skin.

### Disclosure of the Invention

### Problems to be Solved by the Invention

Today, life patterns of individuals are diversified into various kinds, and people seek cosmetics having finely-tuned functions tailored to diversified life scenes.

Under such circumstances, the present inventors have conducted a hearing survey of about 4,700 women to investigate when women feel dullness of bare skin (details will be described hereinbelow). In the survey, about 60% of the women answered that they felt dullness of bare skin more often in the evening. In case where foundation was applied to the skin, it filled the sulcus cutis (i.e., fine skin furrows) under the effect of sebum or the like after a lapse of time, and an area where the foundation remained at high density and a reddish area where bare skin was exposed tended to coexist on the skin. Regarding dullness of bare skin, women are thought to feel dullness of bare skin in the evening, when they are tired from their work, mainly from a psychological aspect. Taking into consideration the psychological aspect and uneven covering of the skin with a foundation after a lapse of time, cosmetic users' skin dullness developed at a specific time in the day is considered to be substantially mitigated by providing means that can cover skin dullness when such uneven covering occurs.

For example, many working women put on their make-up before going to the office in the morning. They often have chances to fix their make-up at lunch break, and they usually leave the office in the evening. At the time of fixing make-up at lunch break, uneven covering of the skin with the applied foundation is thought to occur due to secretion of sebum or the like after morning making-up. To cope with uneven covering, a first conceivable and preferable approach is a cosmetic product containing means for masking skin dullness, the means starting to work at lunch break. Similarly, a second conceivable and preferable approach includes fixing make-up with an additional foundation which contains means for masking skin dullness, the means starting to work in the evening when uneven coverage of the skin with the additional foundation may occur.

Thus, an object of the present invention is to provide a make-up cosmetic composition (e.g., a foundation) which contains latent means for masking skin dullness at the time of application thereof to the skin, the means gradually working a few hours after application.

### Means for Solving the Problems

As means for masking skin dullness, i.e., decreased brightness of the skin, with a foundation or a similar material, incorporation of an interference pearlescent pigment, which has a brightness higher than that of a simple pigment, into such a cosmetic product, is a suitable approach. However, when titanated mica, which is a typical interference pearlescent pigment, is not subjected to any treatment and incorporated into foundation or the like, the "luster" (also called "excessive shining") intrinsic to titanated mica is excessively noticeable, and a cosmetic product containing titanated mica is not suited for use in everyday life. However, as described above, it is difficult for a foundation having a brightness of a daily use level to sufficiently mask skin dullness when the foundation is present unevenly on the skin after a lapse of time. The present inventors have thought that an intrinsic property "luster" of the aforementioned titanated mica can be suitably employed for masking skin dullness of an exposed skin area which occurs due to unevenness of foundation on the skin.

Under such circumstances, the present inventors have conducted further studies, and have found that through incorporation, into a cosmetic composition, of an interference pearlescent pigment combined with a specific material (which hereinafter may be referred to as a "composite pearlescent pigment") in a specific amount, the "luster" of the interference pearlescent pigment remains masked at the time of making up, but the intrinsic "luster" of pearlescent pigment as a base material is developed during progress of uneven distribution of the cosmetic composition (e.g., foundation) occurring via contact between the composite pearlescent pigment and sebum or the like, whereby skin dullness of the exposed skin can be masked. The inventors have also found that through incorporation of spherical powder particles in large amounts and high-viscosity oil into a cosmetic composition, skin dullness can be more effectively masked, the cosmetic composition can be readily applied uniformly, and caking (i.e., cohesion of powder particles to form agglomerates) can be prevented. The present invention has been accomplished on the basis of these findings.

Accordingly, the present invention provides a solid powder cosmetic composition comprising the following ingredients (1) to (3) (hereinafter may be referred to as the cosmetic composition of the present invention):
(1) an interference pearlescent pigment having a reflectance of 30% or higher to visible light having a wavelength of 540 to 580 nm, which has been coated with one or more materials selected from among polymethylmethacrylate, silicon dioxide, platy barium sulfate, zinc oxide, and aluminum chloride, in an amount of 2 to 8 mass%;
(2) spherical powder particles in an amount of 8 to 30 mass%; and
(3) an oil having a viscosity of 500 to 100,000 cps, as measured by means of a B-type viscometer at 30°C, in an amount of 1.5 to 5 mass% (hereinafter may be referred to as a high-viscosity oil);
the amounts being on the basis of the total amount of the composition.

One embodiment of the cosmetic composition of the present invention is a solid powder cosmetic composition for preventing skin dullness.

### Effects of the Invention

The present invention provides a solid powder cosmetic composition which can effectively mask skin dullness emerging on the skin with the lapse of time.

### Brief Description of the Drawings

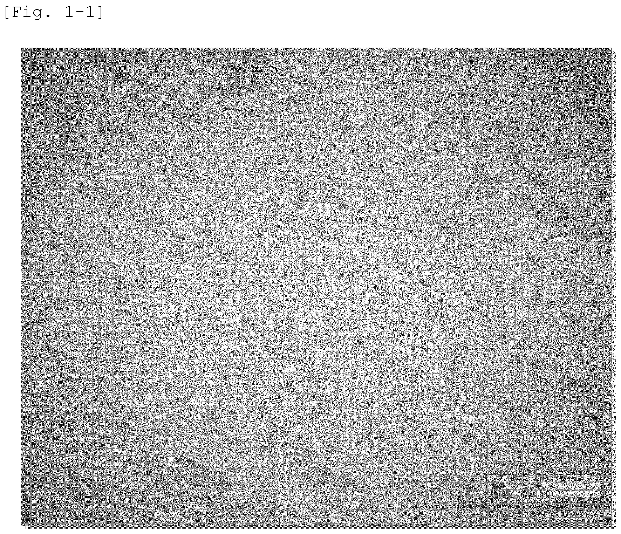

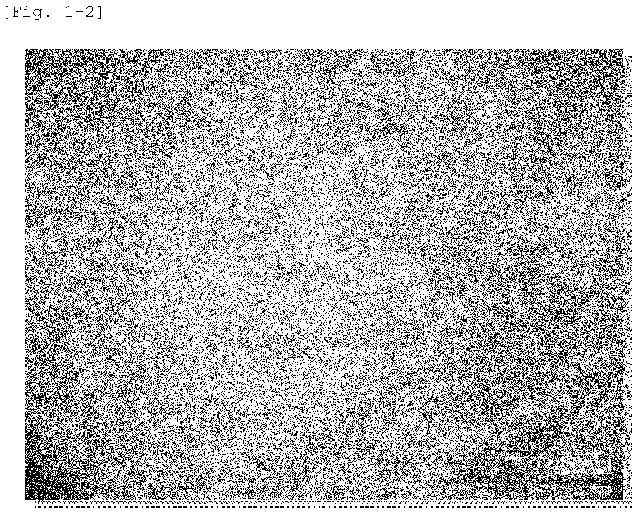

[Fig. 1-1] A microscopic image of skin surface immediately after applying make-up.
[Fig. 1-2] A microscopic image of skin surface a period of time after applying make-up.

### Modes for Carrying Out the Invention

### <Ingredients of the cosmetic composition of the present invention>

### (1) Composite pearlescent pigment

The composite pearlescent pigment employed in the present invention is produced by combining an interference pearlescent pigment having a reflectance of 30% or higher with respect to visible light having a wavelength of 540 to 580 nm, with one or more materials selected from among polymethylmethacrylate (PMMA), silicon dioxide (silica), platy barium sulfate, zinc oxide, and aluminum chloride.

Visible light having a wavelength of 540 to 580 nm is classified as medium-wavelength light. Specific absorption peaks by hemoglobin are observed in this wavelength range, particularly at about 540 nm and about 580 nm. Skin dullness can be substantially masked through inhibition of the specific light absorption by hemoglobin. Therefore, in the present invention, the interference pearlescent pigment serving as a base material of the composite pearlescent pigment preferably has high reflectance to visible light having a wavelength of 540 to 580 nm. Specifically, the interference pearlescent pigment serving as a base material of the composite pearlescent pigment is required to have a reflectance of 30% or higher to visible light having a wavelength falling within the range. No particular limitation is imposed on the base material of the composite pearlescent pigment, so long as the base material has such an optical property. Non-limitative specific examples of the base material include titanated mica (mica substrate coated with titanium dioxide and/or low-valence titanium oxide: also known as mica), iron oxide-coated mica, basic lead carbonate, and bismuth oxychloride. The color of the interference pearlescent pigment may be tuned by varying the thickness of a plate-like particle of the interference pearlescent pigment, and in the case of titanated mica, the thickness of a titanium dioxide layer or a low-valence titanium oxide layer on the mica, etc. In the present invention, the color of the interference pearlescent pigment is suitably gold, yellow, or green. The pearlescent pigment of such a color meets the condition "reflectance to visible light of 540 to 580 nm is 30% or higher." In actual production of cosmetic compositions, the color is particularly preferably gold or yellow, for matching the color of the pigment with the color of the final product. The exemplified interference pearlescent pigments may be produced through a known method. Alternatively, commercial products such as Timiron series (product of Merck) and Flemenco series (product of BASF) may also be used.

The composite pearlescent pigment is a composite powder produced by combining the aforementioned interference pearlescent pigment with one or more materials selected from among polymethylmethacrylate (PMMA), silicon dioxide (silica), platy barium sulfate, zinc oxide, and aluminum chloride (which hereinafter may be referred to as a combining material). The light provided by the interference pearlescent pigment through light interference assumes a characteristic "luster." When the interference pearlescent pigment is directly incorporated into a foundation or the like, the cosmetic product is more likely to exhibit "excessive shining." The "luster" or "excessive shining" may be reduced by combining the pigment with the aforementioned combining material. Also, when the composite pearlescent pigment is in contact with sebum secreted to the skin, the refractive index of the combining material lowers, and the interference color intrinsic to the interference pearlescent pigment is developed. The term "coating" refers to the state in which a large number of microparticles of the combining material are bonded to the surface of a plate-like particle of the interference pearlescent pigment, realizing complete coverage of the interference pearlescent pigment particle. Unless otherwise specified, no particular limitation is imposed on the shape of the microparticles of the combining material. Examples of the particle shape include sphere, plate, and amorphous form. The interference pearlescent pigment may be combined with a combining material through a known method suited for the combining material, such as a mechanochemical process or a chemical process. Alternatively, commercial products such as Excel Pearl series (product of Miyoshi Kasei Inc.) may also be used.

The cosmetic composition of the present invention contains one or more composite pearlescent pigments. The total amount of the composite pearlescent pigments is 2 to 8 mass% with respect to the total amount of the cosmetic composition, preferably 2 to 5 mass%. When the total amount is less than 1 mass% with respect to the amount of the cosmetic composition, difficulty is encountered in completely masking skin dullness, even in the case where the interference color of the interference pearlescent pigment is developed after a lapse of time. When the amount is in excess of 8 mass%, excessive shining attributed to the interference light is strengthened at the time of application of the cosmetic composition and after a lapse of time, whereby the cosmetic product becomes unsuitable for a general use situation.

### (2) Spherical powder particles

No particular limitation is imposed on the material of the spherical powder particles incorporated into the cosmetic composition of the present invention, so long as the material can be incorporated into generally used cosmetic compositions. Examples of the material include polyethylene, polypropylene, polystyrene, polymethylmethacrylate, polyamide resin (Nylon), urethane, silicone resin, silicone rubber, silicone resin-coated rubber, polytetrafluoroethylene, silicon dioxide (silica), styrene-acrylic acid copolymer resin, benzoguanamine resin, and cellulose. The spherical powder particles may be subjected to surface treatment. Examples of the material for the surface treatment include a silicone compound, a fluorine-modified silicone compound, a fluorine-containing compound, a higher fatty acid, a higher alcohol, a fatty acid ester, a metal soap, an amino acid, and an alkyl phosphate. The particle size (mean particle size) of the spherical powder particles is preferably 1 to 30 µm in view of sensation of use of the cosmetic composition, particularly preferably 3 to 20 µm. The cosmetic composition of the present invention may contain spherical powder particles singly or in combination of two or more species.

The cosmetic composition of the present invention contains spherical powder particles in an amount of 8 to 30 mass% with respect to the total amount of the cosmetic composition, preferably 10 to 20 mass%. When the amount of spherical powder particles is in excess of 30 mass%, the cosmetic composition (foundation) is likely to fail to be retained on the skin, whereas when the amount is less than 8 mass%, difficulty is encountered in imparting sufficient extendability to the cosmetic product.

### (3) High-viscosity oil

As described above, the high-viscosity oil incorporated into the cosmetic composition of the present invention has "a viscosity of 500 to 100,000 cps, as measured by means of a B-type viscometer at 30°C." Unless otherwise specified, the "viscosity" in the present invention refers to "the viscosity as measured by means of a B-type viscometer at 30°C." The high-viscosity oil preferably has a viscosity of 1,000 to 20,000 cps. When the viscosity is less than 500 cps, difficulty is encountered in imparting good adhesion to the cosmetic product, and the interference color of the cosmetic product attributed to the aforementioned composite pearlescent pigment is likely to be weakened, whereas when the viscosity is in excess of 100,000 cps, caking of the cosmetic product may occur. Examples of the high-viscosity oil include diisostearyl malate (about 2,000 cps) and glyceryl triisostearate (about 6,000 cps). Into the cosmetic composition of the present invention, one or more high-viscosity oils may be incorporated.

The cosmetic composition of the present invention contains the high-viscosity oil in an amount of 1.5 to 5 mass% with respect to the total amount of the cosmetic composition, preferably 2 to 4 mass%. When the amount is in excess of 5 mass%, caking of the cosmetic product may occur, whereas when the amount is less than 1.5 mass%, difficulty is encountered in imparting good adhesion to the cosmetic product, and the interference color of the cosmetic product attributed to the aforementioned composite pearlescent pigment is likely to be weakened.

### (4) Other ingredients

The cosmetic composition of the present invention may further contain other ingredients employable in solid powder cosmetic compositions in accordance with needs, in qualitative and quantitative ranges so as not to obviously impair target effects of the present invention.

Specific examples of other ingredients include powder ingredients other than the aforementioned (1) "composite pearlescent pigment" and (2) "spherical powder particles" (extender pigment, color pigment, white pigment, and interference pearlescent pigment and functional pigment other than the (1) "composite pearlescent pigment"); oils other than the (3) "high-viscosity oil" (higher alcohol, hydrocarbon oil, ester oil, polar oil, silicone oil, volatile oil, etc.); and other ingredients such as water, a surfactant (cationic surfactant, anionic surfactant, amphoteric surfactant, or nonionic surfactant), a lower alcohol, an ultraviolet absorber, a humectant, a blood flow stimulant, a refrigerant, an antiperspirant, an antibacterial agent, a skin rejuvenating agent, an anti-inflammatory agent, vitamins, an antioxidant, an antiseptic, a perfume, and a whitening agent. These ingredients may be incorporated into the cosmetic composition in the above qualitative and quantitative ranges, in accordance with needs.

### <The cosmetic composition of the present invention>

The cosmetic composition of the present invention is a solid powder cosmetic composition and may be produced as foundation, pressed powder, cheek color, eye shadow, etc. Specifically, the composition may be produced through a conventional method for producing solid powder cosmetic compositions under the conditions specified for the ingredients as described above. Typical production examples are given in the Examples hereinbelow. However, no particular limitation is imposed on the production method, and the cosmetic composition of the present invention may be produced through any of a variety of methods; for example, a wet process employing a solvent, a spray drying process, and agitation/mixing by means of a medium mill.

### Examples

The present invention will next be described in more detail by way of examples. Unless otherwise specified, the amounts of ingredients of the products of the Examples and Comparative Examples shown in the tables are represented by mass% with respect to the composition or product to which the ingredients are incorporated.

### [Investigation of actual sensation of skin dullness]

About 4,700 women were investigated in terms of the time when they feel dullness of bare skin. The investigation was conducted in the following manner.

Panelists of the hearing survey were recruited via the web site of Shiseido Company, Ltd. (the present applicant) and answered the query "Do you feel dullness of bare skin at a certain time of the day? Specify the time when you feel dullness of bare skin." The time zone was set to 24 hours starting from 0 AM with intervals of 1 hour. Multiple choices were accepted.

Through the survey, about 50% of the panelists more strongly felt dullness of bare skin around five o'clock in the evening (panelists who reported feeling no dullness of bare skin (about 14%) were excluded).

In addition to the above query, the panelists answered another query "Do you feel dullness of the skin wearing make-up in the evening after applying make-up?" In response to this query, about 80% of the effective answers were "yes."

Based on these results, the present inventors have thought that change in state of bare skin and foundation-coated skin with lapse of time was caused by some factor, and observed such change in state of foundation-coated skin of each of the 50 women panelists in their 20s to 50s.

In a specific procedure, the aforementioned women panelists applied a foundation of a formulation shown in Table 1 below (produced through a conventional method) after waking up. The skin surface of each panelist was magnified and observed under a video-microscope immediately after making up and at lunch break. As a result, in many panelists, the applied foundation filled the sulcus cutis under the effect of sebum or the like after a lapse of time, and on their skin, an area where the foundation remained at high density and a reddish area where bare skin was exposed were likely to be found. Fig. 1-1 is a microscopic image showing the skin surface of a panelist immediately after applying make-up, and Fig. 1-2 shows a microscopic image showing the skin surface of the same panelist at lunch break when typical uneven coverage of the foundation after a lapse of time was observed. These images are also available as color images. As is clear from Fig. 1-2, the foundation filled the sulcus cutis, to cause uneven coverage of the foundation, whereby the crista cutis was exposed.

**[Table 1]**

| Ingredients | Amount (mass%) |
|---|---|
| Fluorine-treated talc | balance |
| Synthetic fluorphlogopite | 10 |
| Barium sulfate | 8 |
| Silicone-treated titanium oxide | 20 |
| Silicone-treated iron oxide (red) | as appropriate |
| Silicone-treated iron oxide (yellow) | as appropriate |
| Silicone-treated iron oxide (black) | as appropriate |
| Vinyl dimethicone/methicone silsesquioxane crosspolymer | 2 |
| Silica | 2 |
| Nylon powder | 2 |
| Zinc oxide | 3 |
| Silica silylate | 2 |
| Chlorphenesin | as appropriate |
| Squalane | 3 |
| Petrolatum | 1 |
| Ethylhexyl methoxycinnamate | 3 |
| Tocopherol | as appropriate |
| Sorbitan sesquiisostearate | as appropriate |
| Total | 100% |

### Test Examples

### (1) Test method

The present invention was evaluated by use of test cosmetic products which were prepared so as to have the compositions shown in Tables 2 to 4. Each test product was subjected to a test for evaluating properties according to the composition thereof. Firstly, the test methods will be described, and then the composition of the test product and the test results will be disclosed.

### (1)-1: Actual use test (immediately after application)

Each test cosmetic product was evaluated by 20 expert women panelists. Specifically, each panelist applied the test cosmetic product to the face at nine in the morning and evaluated the product in terms of (a) "uniformity immediately after application" (absence of uniformity (lower score) to presence of uniformity (higher score)) and (b) "excessive shining" (excessively shining (lower score) to not shining (higher score)) with a score. The scores given by all the panelists were averaged, and the test cosmetic product was evaluated in the above terms by the average scores.

The scores (1 to 5) given by each panelist correspond to very poor (1), slightly poor (2), fair (3), good (4), and excellent (5). The scores were averaged, and the average value was evaluated with the following ratings.

### <Ratings of evaluation>

4 to 5: ○○ (excellent)
3 or more and less than 4: ○ (fair)
2 or more and less than 3: Δ (slightly poor)
1 or more and less than 2: X (very poor)

### (1)-2: Actual use test (with lapse of time)

In the system of the above test (1)-1, the face of each panelist was observed four hours after application of the test cosmetic product so as to check the state of skin dullness. The extent of skin dullness was evaluated with a score (1 to 5). The scores given by all the panelists were averaged, and the test cosmetic product was evaluated in terms of skin dullness emerging with lapse of time by the average scores (face with skin dullness (lower score) to face having a good complexion with no skin dullness (higher score)) .

The scores (1 to 5) given by each panelist correspond to very poor (1), slightly poor (2), fair (3), good (4), and excellent (5). The scores were averaged, and the average value was evaluated with the following ratings.

### <Ratings of evaluation>

4 to 5: ○○ (excellent)
3 or more and less than 4: ○ (fair)
2 or more and less than 3: Δ (slightly poor)
1 or more and less than 2: X (very poor)

### (1)-3: Caking test

The surface of each test solid cosmetic product was gently rubbed with a sponge. After 200 repetitions of rubbing, the surface of the product was observed.

### <Ratings of evaluation>

○: No abnormality was observed on the surface of the product
Δ: The surface of the product hardened in some degree, but the product could be taken with a sponge
X: Surface hardened, and taking the product was impossible

### (2)-1: The influence of the amount of spherical powder particles (silica, vinyl methicone/methicone silsesquioxane crosspolymer, and polymethylmethacrylate)

Table 2 shows the compositions of the test cosmetic products of Examples 1 to 3 and Comparative Examples 1 and 2, and the test results of "uniformity immediately after application" and "skin dullness emerging with lapse of time."

Each test cosmetic product was produced through a method generally employed for preparing powder compact products. Specifically, a powder portion and an oily portion were mixed with agitation by means of a Henschel mixer (product of Mitsui Mining Co., Ltd.) and then the mixture was pulverized by means of a pulverizer (product of Hosokawa Micron Corporation). The product was put into a cosmetic pan and pressed, to thereby produce a test cosmetic product.

**[Table 2]**

| | Ex. 1 | Ex. 2 | Ex. 3 | Comp. Ex. 1 | Comp. Ex. 2 |
|---|---|---|---|---|---|
| Ingredients | Amount (mass%) | | | | |
| Metallic soap-treated talc | balance | balance | balance | balance | balance |
| Synthetic mica | 20 | 20 | 20 | 20 | 20 |
| Silicone-treated sericite | 15 | 15 | 15 | 15 | 15 |
| Platy barium sulfate | 3 | 3 | 3 | 3 | 3 |
| Synthetic wax | 3 | 3 | 3 | 3 | 3 |
| Silica | 5 | - | - | 2 | 10 |
| Vinyl dimethicone/methicone silsesquioxane crosspolymer | 5 | 10 | - | 2 | 15 |
| Polymethylmethacrylate | 5 | 5 | 10 | 2 | 15 |
| Micro titanium oxide | 8 | 8 | 8 | 8 | 8 |
| Titanium oxide | 10 | 10 | 10 | 10 | 10 |
| Red iron oxide | as appropriate | as appropriate | as appropriate | as appropriate | as appropriate |
| Yellow iron oxide | as appropriate | as appropriate | as appropriate | as appropriate | as appropriate |
| Black iron oxide | as appropriate | as appropriate | as appropriate | as appropriate | as appropriate |
| Iron oxide-coated titanated mica | 2 | 2 | 2 | 2 | 2 |
| PMMA-coated titanated mica | 3 | 3 | 3 | 3 | 3 |
| Hyaluronic acid | as appropriate | as appropriate | as appropriate | as appropriate | as appropriate |
| Tranexamic acid | 2 | 2 | 2 | 2 | 2 |
| Chlorphenesin | as appropriate | as appropriate | as appropriate | as appropriate | as appropriate |
| Diisostearyl malate | 3 | 3 | 3 | 3 | 3 |
| Dimethicone | 4 | 4 | 4 | 4 | 4 |
| Ethylhexyl methoxycinnamate | 4 | 4 | 4 | 4 | 4 |
| Sorbitan sesquiisostearate | 1 | 1 | 1 | 1 | 1 |
| Tocopherol | as appropriate | as appropriate | as appropriate | as appropriate | as appropriate |
| Perfume | as appropriate | as appropriate | as appropriate | as appropriate | as appropriate |
| Total | 100 | 100 | 100 | 100 | 100 |

| Evaluation | | | | | |
|---|---|---|---|---|---|
| Uniformity immediately after application | ○○ | ○ | ○ | Δ | X |
| Dullness after a lapse of time | ○○ | ○ | ○ | Δ | X |

The employed materials listed in Table 2 are as follows: "silica" was Sansphere L-51S (product of Dokai Kagaku Kogyo); "vinyl dimethicone/methicone silsesquioxane crosspolymer" was Silicone Powder KSP100 (product of Shin-Etsu Chemical Co., Ltd.); "polymethylmethacrylate" was Ganz Pearl GMX-0810 (product of Ganz Chemical Co., Ltd.); "iron oxide-coated titanated mica" was produced by using Timiron Super Gold (product of Merck) as a base material, and iron oxide pigment (e.g., red iron oxide) as a coating; "polymethylmethacrylate-coated titanated mica" was produced by using Timiron Super Gold (product of Merck) as a base material, and polymethylmethacrylate as a coating; and "diisostearyl malate" was Cosmol 222 (product of Nisshin Seiyu).

Among them, the "polymethylmethacrylate-coated titanated mica" is a composite pearlescent pigment. The "silica," "vinyl dimethicone/methicone silsesquioxane crosspolymer," and "polymethylmethacrylate" are spherical powder particles having a mean particle size of about 5 µm. The "diisostearyl malate" is a high-viscosity oil having a viscosity of about 2,000 cps.

Unless otherwise specified, the same ingredients as disclosed in Table 2 were used to prepare test cosmetic products in the following experiments.

As is clear from Table 2, when the amount of spherical powder particles is smaller than the predetermined value, the uniformity immediately after application and the extent of skin dullness emerging with lapse of time were inferior to those of Examples 1 to 3.

### (2)-2: The influence of composite pearlescent pigments (polymethylmethacrylate-coated titanated mica, zinc oxide-coated titanated mica, and aluminum chloride-coated titanated mica)

Table 3 shows the compositions of the test cosmetic products of Examples 1, and 4 to 7, and Comparative Examples 3 and 4, and the test results of "skin dullness emerging with lapse of time." The test cosmetic products were produced through the same method as employed in production of the test products shown in Table 2.

**[Table 3-1]**

| | Ex. 1 | Ex. 4 | Ex. 5 | Ex. 6 |
|---|---|---|---|---|
| Ingredients | Amount (mass%) | | | |
| Metallic soap-treated talc | balance | balance | balance | balance |
| Synthetic mica | 20 | 20 | 20 | 20 |
| Silicone-treated sericite | 15 | 15 | 15 | 15 |
| Platy Barium sulfate | 3 | 3 | 3 | 3 |
| Synthetic wax | 3 | 3 | 3 | 3 |
| Silica | 5 | 5 | 5 | 5 |
| Vinyl dimethicone/methicone silsesquioxane crosspolymer | 5 | 5 | 5 | 5 |
| Polymethylmethacrylate | 5 | 5 | 5 | 5 |
| Micro titanium oxide | 8 | 8 | 8 | 8 |
| Titanium oxide | 10 | 10 | 10 | 10 |
| Red iron oxide | as appropriate | as appropriate | as appropriate | as appropriate |
| Yellow iron oxide | as appropriate | as appropriate | as appropriate | as appropriate |
| Black iron oxide | as appropriate | as appropriate | as appropriate | as appropriate |
| Iron oxide-coated titanated mica | 2 | 2 | 2 | 2 |
| PMMA-coated titanated mica | 3 | 5 | 8 | - |
| Zinc oxide-coated titanated mica | - | - | - | 3 |
| Aluminum chloride-coated titanated mica | - | - | - | - |
| Hyaluronic acid | as appropriate | as appropriate | as appropriate | as appropriate |
| Tranexamic acid | 2 | 2 | 2 | 2 |
| Chlorphenesin | as appropriate | as appropriate | as appropriate | as appropriate |
| Diisostearyl malate | 3 | 3 | 3 | 3 |
| Dimethicone | 4 | 4 | 4 | 4 |
| Ethylhexyl methoxycinnamate | 4 | 4 | 4 | 4 |
| Sorbitan sesquiisostearate | 1 | 1 | 1 | 1 |
| Tocopherol | as appropriate | as appropriate | as appropriate | as appropriate |
| Perfume | as appropriate | as appropriate | as appropriate | as appropriate |
| Total | 100 | 100 | 100 | 100 |

| Evaluation | | | | |
|---|---|---|---|---|
| Dullness after a lapse of time | ○○ | ○ | ○ | ○ |

**[Table 3-2]**

| | Ex. 7 | Comp. Ex. 3 | Comp. Ex. 4 |
|---|---|---|---|
| Ingredients | Amount (mass%) | | |
| Metallic soap-treated talc | balance | balance | balance |
| Synthetic mica | 20 | 20 | 20 |
| Silicone-treated sericite | 15 | 15 | 15 |
| Platy Barium sulfate | 3 | 3 | 3 |
| Synthetic wax | 3 | 3 | 3 |
| Silica | 5 | 5 | 5 |
| Vinyl dimethicone/methicone silsesquioxane crosspolymer | 5 | 5 | 5 |
| Polymethylmethacrylate | 5 | 5 | 5 |
| Micro titanium oxide | 8 | 8 | 8 |
| Titanium oxide | 10 | 10 | 10 |
| Red iron oxide | as appropriate | as appropriate | as appropriate |
| Yellow iron oxide | as appropriate | as appropriate | as appropriate |
| Black iron oxide | as appropriate | as appropriate | as appropriate |
| Iron oxide-coated titanated mica | 2 | 2 | 2 |
| PMMA-coated titanated mica | - | 1 | 15 |
| Zinc oxide-coated titanated mica | - | - | - |
| Aluminum chloride-coated titanated mica | 3 | - | - |
| Hyaluronic acid | as appropriate | as appropriate | as appropriate |
| Tranexamic acid | 2 | 2 | 2 |
| Chlorphenesin | as appropriate | as appropriate | as appropriate |
| Diisostearyl malate | 3 | 3 | 3 |
| Dimethicone | 4 | 4 | 4 |
| Ethylhexyl methoxycinnamate | 4 | 4 | 4 |
| Sorbitan sesquiisostearate | 1 | 1 | 1 |
| Tocopherol | as appropriate | as appropriate | as appropriate |
| Perfume | as appropriate | as appropriate | as appropriate |
| Total | 100 | 100 | 100 |

| Evaluation | | | |
|---|---|---|---|
| Dullness after a lapse of time | ○ | Δ | X |

Regarding the employed materials listed in Table 3, the base material of polymethylmethacrylate-coated titanated mica and that of zinc oxide-coated titanated mica was Timiron Super Gold (product of Merck). The coated titanated mica was produced by coating the base material with polymethylmethacrylate or zinc oxide through a conventional method. Aluminum chloride-coated titanated mica was Excel Pearl (gold) (product of Miyoshi Kasei Inc.).

As is clear from Table 3, skin dullness emerging with lapse of time was favorably masked, when each of the composite pearlescent pigments was incorporated in an amount falling within the scope of the invention. However, the skin dullness was not suitably masked, when the amount was excessively large or small.

### (2)-3: The influence of high-viscosity oil (diisostearyl malate and glyceryl triisostearate)

Table 4 shows the compositions of the test cosmetic products of Examples 1 and 8 and Comparative Examples 5 and 6, and the test results of "uniformity immediately after application" and "caking." The test cosmetic products were produced through the same method as employed in production of the test products shown in Table 2.

**[Table 4]**

| | Ex. 1 | Ex. 8 | Comp. Ex. 5 | Comp. Ex. 6 |
|---|---|---|---|---|
| Ingredients | Amount (mass%) | | | |
| Metallic soap-treated talc | balance | balance | balance | balance |
| Synthetic mica | 20 | 20 | 20 | 20 |
| Silicone-treated sericite | 15 | 15 | 15 | 15 |
| Platy Barium sulfate | 3 | 3 | 3 | 3 |
| Synthetic wax | 3 | 3 | 3 | 3 |
| Silica | 5 | 5 | 5 | 5 |
| Vinyl dimethicone/methicone silsesquioxane crosspolymer | 5 | 5 | 5 | 5 |
| Polymethylmethacrylate | 5 | 5 | 5 | 5 |
| Micro titanium oxide | 8 | 8 | 8 | 8 |
| Titanium oxide | 10 | 10 | 10 | 10 |
| Red iron oxide | as appropriate | as appropriate | as appropriate | as appropriate |
| Yellow iron oxide | as appropriate | as appropriate | as appropriate | as appropriate |
| Black iron oxide | as appropriate | as appropriate | as appropriate | as appropriate |
| Iron oxide-coated titanated mica | 2 | 2 | 2 | 2 |
| PMMA-coated titanated mica | 3 | 3 | 3 | 3 |
| Hyaluronic acid | as appropriate | as appropriate | as appropriate | as appropriate |
| Tranexamic acid | 2 | 2 | 2 | 2 |
| Chlorphenesin | as appropriate | as appropriate | as appropriate | as appropriate |
| Diisostearyl malate | 3 | - | 1 | 8 |
| Glyceryl triisostearate | - | 3 | - | - |
| Dimethicone | 4 | 4 | 6 | - |
| Ethylhexyl methoxycinnamate | 4 | 4 | 4 | 3 |
| Sorbitan sesquiisostearate | 1 | 1 | 1 | 1 |
| Tocopherol | as appropriate | as appropriate | as appropriate | as appropriate |
| Perfume | as appropriate | as appropriate | as appropriate | as appropriate |
| Total | 100 | 100 | 100 | 100 |

| Evaluation | | | | |
|---|---|---|---|---|
| Uniformity immediately after application | ○○ | ○○ | X | X |
| Caking | ○ | ○ | ○ | X |

In Table 4, "glyceryl triisostearate" is a high-viscosity oil having a viscosity of about 6,000 cps. A commercial product thereof, Sun Espol GTI(S) (product of Taiyo Kagaku Co., Ltd.), was used as "glyceryl triisostearate."

As is clear from Table 4, uniformity immediately after application was lacking, when the amount of high-viscosity oil was excessively small. In contrast, when the amount was excessively large, attention should be paid to uniformity immediately after application and occurrence of caking.

## Claims

1. A solid powder cosmetic composition comprising the following ingredients (1) to (3):
(1) an interference pearlescent pigment having a reflectance of 30% or higher to visible light having a wavelength of 540 to 580 nm, which has been coated with one or more materials selected from among polymethylmethacrylate, silicon dioxide, platy barium sulfate, zinc oxide, and aluminum chloride, in an amount of 2 to 8 mass%;
(2) spherical powder particles in an amount of 8 to 30 mass%; and
(3) an oil having a viscosity of 500 to 100,000 mPa.s, as measured by means of a B-type viscometer at 30°C, in an amount of 1.5 to 5 mass%;
the amounts being on the basis of the total amount of the composition.

2. The solid powder cosmetic composition according to claim 1, wherein the interference pearlescent pigment is titanated mica assuming a color of gold, yellow, or green.

3. The solid powder cosmetic composition according to claim 1, wherein the interference pearlescent pigment is titanated mica assuming a color of gold or yellow.

4. The solid powder cosmetic composition according to any of claims 1 to 3, wherein the material with which the interference pearlescent pigment is coated is polymethylmethacrylate.

5. The solid powder cosmetic composition according to any of claims 1 to 4, which is a solid powder cosmetic composition for masking skin dullness.

## Patentansprüche

1. Feste, pulverförmige kosmetische Zusammensetzung, die die folgenden Bestandteile (1) bis (3) umfasst:
(1) ein Interferenz-Perlglanzpigment, das ein Reflexionsvermögen von 30 % oder höher bezüglich sichtbarem Licht mit einer Wellenlänge von 540 bis 580 nm aufweist, das mit einem oder mehreren Material(ien), die aus Polymethylmethacrylat, Siliciumdioxid, plättchenförmigem Bariumsulfat, Zinkoxid und Aluminiumchlorid ausgewählt sind, in einer Menge von 2 bis 8 Massen-% beschichtet worden ist,
(2) kugelförmige pulverförmige Partikel in einer Menge von 8 bis 30 Massen-% und
(3) ein Öl, das eine Viskosität von 500 bis 100.000 mPa·s gemäß Messung mittels eines Viskosimeters vom B-Typ bei 30 °C aufweist, in einer Menge von 1,5 bis 5 Massen-%,
wobei die Mengen sich auf die Gesamtmenge der Zusammensetzung beziehen.

2. Feste, pulverförmige kosmetische Zusammensetzung nach Anspruch 1, wobei das Interferenz-Perlglanzpigment titanisierter Glimmer ist, der eine Farbe von golden, gelb oder grün annimmt.

3. Feste, pulverförmige kosmetische Zusammensetzung nach Anspruch 1, wobei das Interferenz-Perlglanzpigment titanisierter Glimmer ist, der eine Farbe von golden oder gelb annimmt.

4. Feste, pulverförmige kosmetische Zusammensetzung nach einem der Ansprüche 1 bis 3, wobei das Material, mit dem das Interferenz-Perlglanzpigment beschichtet ist, Polymethylmethacrylat ist.

5. Feste, pulverförmige kosmetische Zusammensetzung nach einem der Ansprüche 1 bis 4, bei der es sich um eine feste, pulverförmige kosmetische Zusammensetzung zum Abdecken einer Mattheit der Haut handelt.

## Revendications

1. Composition cosmétique sous forme de poudre solide comprenant les ingrédients (1) à (3) suivants :
(1) un pigment perlé d'interférence ayant une réflectance supérieure ou égale à 30 % à la lumière visible ayant une longueur comprise entre 540 et 580 nm, qui a été revêtu par au moins un matériau choisi parmi le polyméthylméthacrylate, le dioxyde de silicium, le sulfate de baryum lamellaire, l'oxyde de zinc et le chlorure d'aluminium, en une quantité comprise entre 2 et 8 % en masse ;
(2) des particules de poudre sphériques en une quantité comprise entre 8 et 30 % en masse ; et
(3) une huile ayant une viscosité comprise entre 500 et 100 000 mPa.s, telle que mesurée au moyen d'un viscosimètre de type B à 30 °C, en une quantité comprise entre 1,5 et 5 % en masse ;
les quantités étant sur la base de la quantité totale de la composition.

2. Composition cosmétique sous forme de poudre solide selon la revendication 1, dans laquelle le pigment perlé d'interférence est le mica à base de titane prenant une couleur or, jaune ou verte.

3. Composition cosmétique sous forme de poudre solide selon la revendication 1, dans laquelle le pigment perlé d'interférence est le mica à base de titane prenant une couleur or ou jaune.

4. Composition cosmétique sous forme de poudre solide selon l'une quelconque des revendications 1 à 3, dans laquelle le matériau avec lequel est revêtu le pigment perlé d'interférence est le polyméthylméthacrylate.

5. Composition cosmétique sous forme de poudre solide selon l'une quelconque des revendications 1 à 4, qui est une composition cosmétique sous forme de poudre solide permettant de masquer le teint terne.
